# EUROPEAN PATENT APPLICATION

(11) **EP 4 623 918 A1**
(43) Date of publication of application: **01.10.2025**
(21) Application number: 23894537.2
(22) Date of filing: 20.11.2023
(51) Int. Cl.: A61K 31/685, A61P 25/16, A61P 25/18, A61P 25/24, A61P 25/28, A61P 43/00

(54) **AGENT FOR INCREASING BRAIN PLASMALOGEN, AND USE OF ORAL COMPOSITION AND COMPOUND FOR INCREASING BRAIN PLASMALOGEN**

(30) Priority: 21.11.2022 JP 2022185424
(71) Applicant: Institute of Rheological Function of Food Co., Ltd., Fukuoka 811-2501 (JP); Kyushu University, National University Corporation, Nishi-ku Fukuoka-shi Fukuoka 819-0395 (JP)
(72) Inventor: FUJINO Takehiko, Fukuoka-shi, Fukuoka 813-0043 (JP); MAWATARI Shiro, Fukuoka-shi, Fukuoka 813-0016 (JP); NIWASE Shamim, Fukuoka-shi, Fukuoka 810-0054 (JP); HONSHO Masanori, Fukuoka-shi, Fukuoka 819-0395 (JP); NAKASHIMA Kinichi, Fukuoka-shi, Fukuoka 819-0395 (JP); NAKASHIMA Hideyuki, Fukuoka-shi, Fukuoka 819-0395 (JP); OKAUCHI Tatsuo, Kitakyushu-shi, Fukuoka 804-8550 (JP)
(74) Representative: V.O.
(86) International application number: PCT/JP2023/041563
(87) International publication number: WO 2024/111529

(57) **Abstract**

A brain plasmalogen increaser includes a compound of Formula (I): 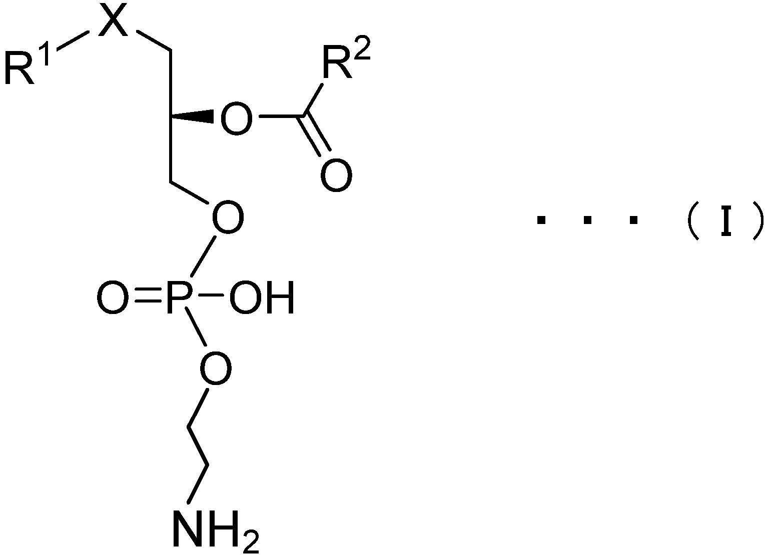 (where, in Formula (I), X represents a carbon atom or an oxygen atom, R¹ represents a saturated aliphatic hydrocarbon group optionally containing a ring structure, and R² represents a saturated or unsaturated aliphatic hydrocarbon group having 10 to 30 carbon atoms, optionally containing a substituent that is an alkyl group having 1 to 4 carbon atoms or an alkoxy group having 1 to 4 carbon atoms), a racemate of the compound, or a salt of the compound or the racemate.

## Description

### Technical Field

The present disclosure relates to a brain plasmalogen increaser, an oral composition for increasing brain plasmalogen, and use of a compound.

### Background Art

Plasmalogens are a type of phospholipid having antioxidant action, and are glycerophospholipids. Plasmalogens are present in all mammalian tissues, and account for about 18% of phospholipids in the human body.

Since most plasmalogens are bound to polyunsaturated fatty acids such as docosahexaenoic acid and arachidonic acid, they are involved in, for example, storing polyunsaturated fatty acids, and releasing secondary messengers of intercellular signals such as prostaglandin and leukotrienes produced from the polyunsaturated fatty acids. Plasmalogens are also involved in, for example, cell fusion and ion transport. Since vinyl ether bonds (alkenyl bonds) of plasmalogens are particularly sensitive to oxidative stress, plasmalogens also have an antioxidant function for cells.

In addition, plasmalogens are known to have, for example, an action to promote neurogenesis, an action to suppress neuroinflammation due to lipopolysaccharides (LPS), and an action to suppress accumulation of amyloid β (Aβ) protein in the brain.

Plasmalogens are known to be decreased in neurological diseases such as dementia, Parkinson's disease, depression, and schizophrenia. For example, Non Patent Literature 1 has reported that ethanolamine-type plasmalogen is highly significantly decreased in the frontal lobe and the hippocampus in the brain in Alzheimer's disease. Therefore, an effect to prevent and ameliorate the aforementioned diseases can be expected by increasing the plasmalogen that has been decreased. For example, Patent Literature 1 discloses a drug against central nervous system inflammation, containing plasmalogen. Non Patent Literature 2 has reported that a randomized double-blind clinical trial was carried out by oral administration of plasmalogen extracted from scallop mantle to human patients with mild Alzheimer's disease or mild cognitive impairment, and that the result of the trial strongly suggested improvement in the cognitive function in mild Alzheimer's disease. Non-Patent Literature 3 has shown that learning ability and memory capability of mice to which plasmalogen extracted from scallops was orally administered, were enhanced.

### Citation List

### Patent Literature

Patent Literature 1: International Publication No. WO 2012/039472

### Non Patent Literature

Non Patent Literature 1: Z. Guan, et al., Decrease and Structural Modifications of Phosphatidylethanolamine Plasmalogen in the Brain with Alzheimer Disease, J Neuropathol Exp Neurol, 58 (7), 740-747, 1999
Non Patent Literature 2: T. Fujino, et al., Efficacy and Blood Plasmalogen Changes by Oral Administration of Plasmalogen in Patients with Mild Alzheimer's Disease and Mild Cognitive Impairment: A Multicenter, Randomized, Double-blind, Placebo-controlled Trial, EBioMedicine, 17: 199-205, 2017
Non-Patent Literature 3: MS. Hossain, et al., Plasmalogens, the Vinyl Ether-Linked Glycerophospholipids, Enhance Learning and Memory by Regulating Brain-Derived Neurotrophic Factor, Front. Cell Dev. Biol. 10:828282, 2022

### Summary of Invention

### Technical Problem

In view of the above circumstances, an objective of the present disclosure is to provide a brain plasmalogen increaser that is capable of increasing plasmalogen in the brain, an oral composition for increasing brain plasmalogen, and use of a compound.

### Solution to Problem

A brain plasmalogen increaser according to a first aspect of the present disclosure includes a compound of Formula (I): 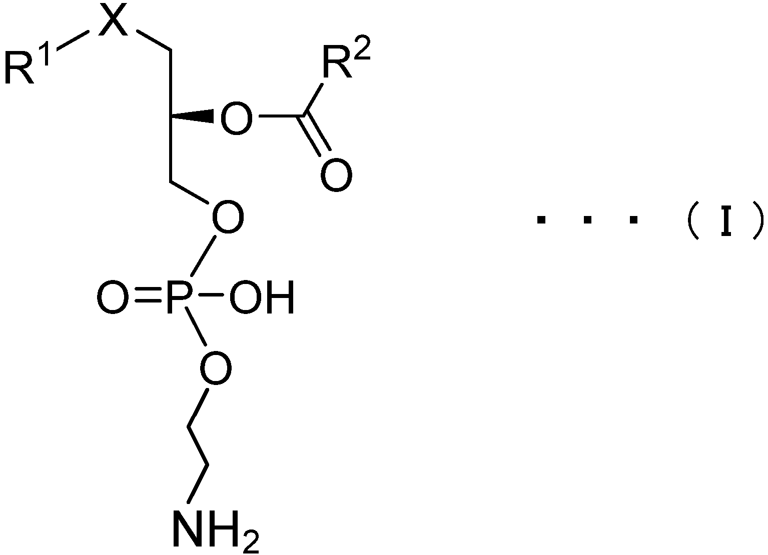 (where, in Formula (I), X represents a carbon atom or an oxygen atom, R¹ represents a saturated aliphatic hydrocarbon group optionally containing a ring structure, and R² represents a saturated or unsaturated aliphatic hydrocarbon group having 10 to 30 carbon atoms, optionally containing a substituent that is an alkyl group having 1 to 4 carbon atoms or an alkoxy group having 1 to 4 carbon atoms), a racemate of the compound, or a salt of the compound or the racemate.

An oral composition for increasing brain plasmalogen according to a second aspect of the present disclosure includes a compound of Formula (I): 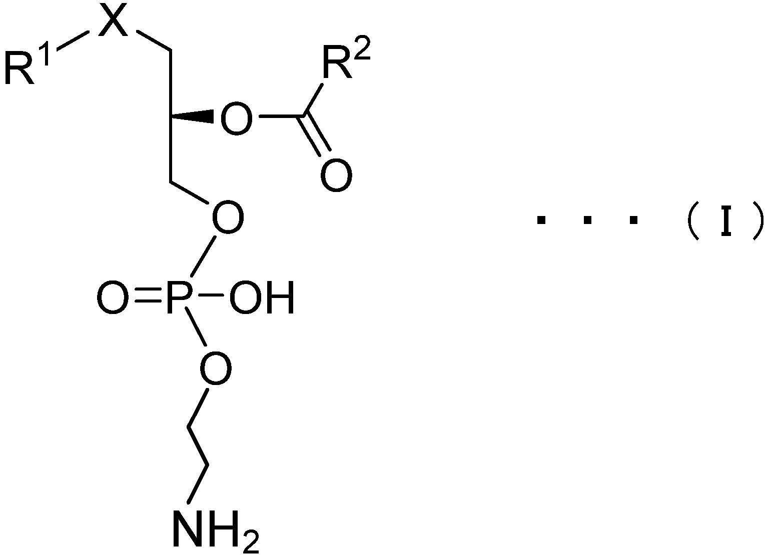 (where, in Formula (I), X represents a carbon atom or an oxygen atom, R¹ represents a saturated aliphatic hydrocarbon group optionally containing a ring structure, and R² represents a saturated or unsaturated aliphatic hydrocarbon group having 10 to 30 carbon atoms, optionally containing a substituent that is an alkyl group having 1 to 4 carbon atoms or an alkoxy group having 1 to 4 carbon atoms), a racemate of the compound, or a salt of the compound or the racemate.

X may represent an oxygen atom, R¹ may represent an alkyl group having 18 carbon atoms, and R² may represent an unsaturated aliphatic hydrocarbon group.

R², when defined as R²COOH, may represent a monovalent group corresponding to arachidonic acid.

Use of a compound according to a third aspect of the present disclosure is use of a compound of Formula (I): 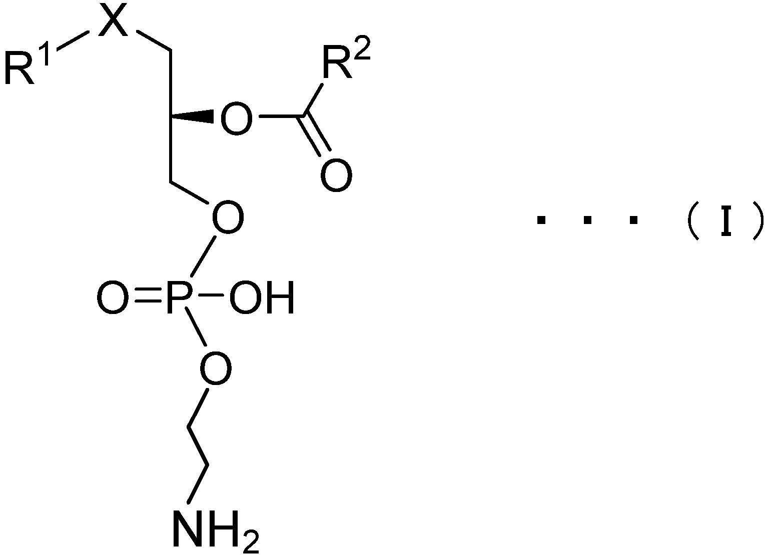 for producing a brain plasmalogen increaser or an oral composition for increasing brain plasmalogen, a racemate of the compound, or a salt of the compound or the racemate. In Formula (I), X represents a carbon atom or an oxygen atom, R¹ represents a saturated aliphatic hydrocarbon group optionally containing a ring structure, and R² represents a saturated or unsaturated aliphatic hydrocarbon group having 10 to 30 carbon atoms, optionally containing a substituent that is an alkyl group having 1 to 4 carbon atoms or an alkoxy group having 1 to 4 carbon atoms.

### Advantageous Effects of Invention

The present disclosure enables plasmalogen in the brain to be increased.

### Brief Description of Drawings

FIG. 1A is a diagram illustrating the concentration of plasmalogen in cerebral cortex tissue lysate according to Example 2;
FIG. 1B is a diagram illustrating the concentration of plasmalogen in cerebral cortex tissue lysate according to Example 2, in which the sn-1 position of the plasmalogen is a saturated aliphatic hydrocarbon group having 18 carbon atoms (C18:0); and
FIG. 2 is a diagram illustrating the concentration of plasmalogen in cerebral cortex tissue lysate according to Example 3.

### Description of Embodiments

Embodiments according to the present disclosure are described below with reference to the drawings. The present disclosure is not limited by the following embodiments and drawings. In the following embodiments, expressions such as "have", "include", or "contain" also encompass the meaning of "consist of" or "be constituted of".

### Embodiments

A brain plasmalogen increaser according to the present embodiment includes a compound of Formula (I) below, a racemate of the compound, or a salt of the compound or the racemate.

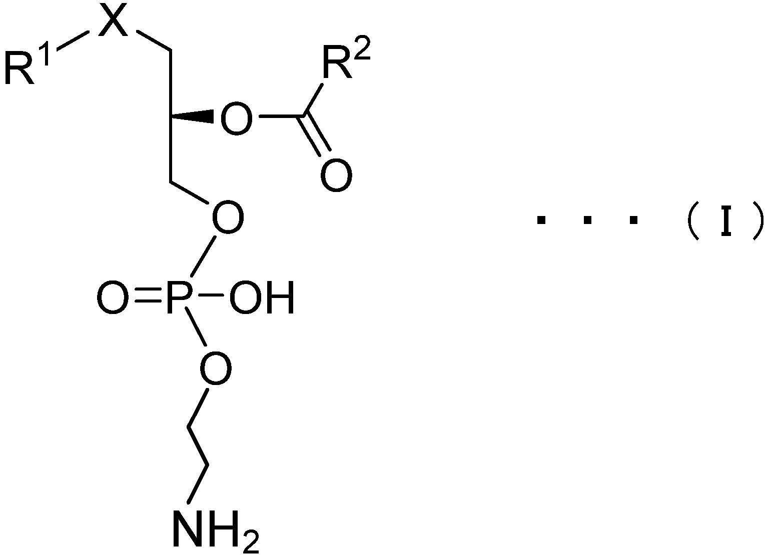

In Formula (I), X represents a carbon atom or an oxygen atom. X preferably represents an oxygen atom. R¹ represents a saturated aliphatic hydrocarbon group optionally containing a ring structure. The number of carbon atoms of the saturated aliphatic hydrocarbon group is not limited. The number of carbon atoms of the saturated aliphatic hydrocarbon group may be, for example, 10 to 30, 10 to 25, or 10 to 20. The number of carbon atoms constituting the ring structure is, for example, 3 to 6. The ring structure may be contained as a cycloalkylene group in R¹, or as a cycloalkyl group in R¹. The number of carbon atoms constituting the ring structure is preferably 3. The saturated aliphatic hydrocarbon group may contain the ring structure, as a monovalent group, at the terminus of R¹. R¹ preferably represents an alkyl group having 18 carbon atoms.

R² represents a saturated or unsaturated aliphatic hydrocarbon group having 10 to 30 carbon atoms, optionally containing a substituent that is an alkyl group having 1 to 4 carbon atoms or an alkoxy group having 1 to 4 carbon atoms. R² preferably represents an unsaturated aliphatic hydrocarbon group. For example, R², when defined as R²COOH, represents a monovalent group corresponding to ω-3 fatty acids, ω-6 fatty acids, ω-7 fatty acids, ω-9 fatty acids, and ω-10 fatty acids. R², when defined as R²COOH, preferably represents a monovalent group corresponding to arachidonic acid, docosahexaenoic acid, oleic acid, eicosapentaenoic acid, or linoleic acid.

The salt of the compound of Formula (I) above and the salt of the racemate of the compound are not limited provided that the salts are pharmacologically acceptable salts, and may be either an acidic salt or a basic salt. Examples of the salts include alkali metal salts such as lithium salt, sodium salt, and potassium salt; alkaline earth metal salts such as magnesium salt and calcium salt; inorganic acid salts such as hydrochloride, hydrobromide, sulfate, nitrate, oxalate, and phosphate; and organic acid salts such as acetate, propionate, hexanoate, cyclopentane propionate, glycolate, pyruvate, lactate, malonate, succinate, malate, fumarate, tartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, camphorsulfonate, glucoheptanoate, 3-phenylpropionate, trimethylacetate, tertiary butyl acetate, lauryl sulfate, gluconate, glutamate, hydroxynaphthoate, salicylate, stearate, trifluoroacetate (TFA), maleate, and muconate.

Preferably, the brain plasmalogen increaser according to the present embodiment includes a compound of the Formula below, a racemate of the compound, or a salt of the compound or the racemate.

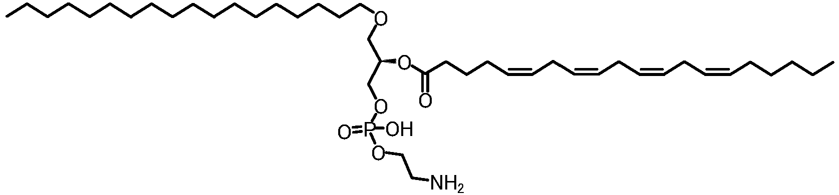

The compound according to the present embodiment can be synthesized by a known method. For example, arachidonic acid is reacted with tert-butyl(2-((tert-butoxy((R)-2-hydroxy-3-(octadecyloxy)propoxy)phosphoryl)oxy)ethyl)carbamate using a condensing agent. The resulting product is deprotected by allowing a strong acid such as trifluoroacetic acid to act on the product, to obtain a compound according to the present embodiment. The condensing agent is not limited, and may be, for example, 1-(3-dimethylaminopropyl)-3-ethylcarbodiimide (EDC) hydrochloride, dicyclohexylcarbodiimide (DCC), HATU, HBTU, TATU, TBTU, and diphenylphosphoryl azide (DPPA). For promoting the dehydration condensation reaction by the condensing agent, a nucleophile such as dimethylaminopyridine (DMAP) may be used.

The brain plasmalogen increaser according to the present embodiment contains the compound of Formula (I) above, the racemate of the compound, or the salt of the compound or the racemate (hereinafter also referred to as "compound and the like"), as effective components, and may also contain other pharmacologically acceptable components. Specific examples of the other pharmacologically acceptable components include excipients, lubricants, binders, disintegrators, solvents, solubilizers, suspending agents, tonicity agents, buffers, soothing agents, and the like. As necessary, additives such as preservatives, antioxidants, coloring agents, sweeteners, or the like may be added to the brain plasmalogen increaser.

Examples of the excipients include lactose, white sugar, D-mannitol, starch, crystalline cellulose, light anhydrous silicic acid, and the like. Examples of the lubricants include magnesium stearate, calcium stearate, talc, colloidal silica, and the like. Examples of the binders include crystalline cellulose, white sugar, D-mannitol, dextrin, hydroxypropylcellulose, hydroxypropylmethylcellulose, polyvinylpyrrolidone, and the like. Examples of the disintegrants include starch, carboxymethyl cellulose, carboxymethyl cellulose calcium, croscarmellose sodium, carboxymethyl starch sodium, and the like.

Examples of the solvents include water for injection, alcohol, propylene glycol, macrogol, and the like. Examples of the solubilizers include polyethylene glycol, propylene glycol, D-mannitol, benzyl benzoate, ethanol, trisaminomethane, cholesterol, triethanolamine, sodium carbonate, sodium citrate, and the like. The suspending agent is a surfactant, a hydrophilic polymer, or the like. Examples thereof include stearyltriethanolamine, sodium lauryl sulfate, lauryl aminopropionic acid, lecithin, benzalkonium chloride, benzethonium chloride, glycerol monostearate, polyvinyl alcohol, polyvinylpyrrolidone, sodium carboxymethylcellulose, methylcellulose, hydroxymethylcellulose, hydroxyethylcellulose, hydroxypropylcellulose, or the like.

Examples of the tonicity agents include sodium chloride, glycerin, D-mannitol, and the like. Examples of the buffering agents include phosphate, acetate, carbonate, citrate buffers, and the like. Examples of the soothing agents include benzyl alcohol, and the like. Examples of the preservatives include paraoxybenzoic acid esters, chlorobutanol, benzyl alcohol, phenethyl alcohol, dehydroacetic acid, sorbic acid, and the like. Examples of the antioxidants include sulfite, ascorbic acid, and the like.

The brain plasmalogen increaser according to the present embodiment increases, as described in the examples below, plasmalogens in the brain from before the administration of the brain plasmalogen increaser. Plasmalogens belong to a subclass specific to glycerophospholipids characterized by the presence of a vinyl ether bond at the sn-1 position, and an ester bond at the sn-2 position, of the glycerol backbone. The plasmalogen in the brain increased by the brain plasmalogen increaser is not limited provided that the plasmalogen is a glycerophospholipid typically classified as plasmalogen. Examples of the plasmalogens include ethanolamine plasmalogen (PlsEtn), choline plasmalogen (PlsCho), inositol plasmalogen, serine plasmalogen, and the like. Pls has a structure in which -CH₂CH₂-NH₂ is bound to the oxygen atom that is bound to the phosphorus at the sn-3 position of the glycerol backbone. PlsCho has a structure in which -CH₂CH₂N(CH₃)₃ is bound to the oxygen atom that is bound to the phosphorus at the sn-3 position of the glycerol backbone.

The brain plasmalogen increaser according to the present embodiment increases plasmalogen in the brain, and is therefore useful for the prevention and treatment of symptoms or diseases caused by a decrease in plasmalogen in the brain. Examples of the diseases caused by the decrease in plasmalogen in the brain include dementia, Alzheimer's disease, Parkinson's disease, depression, and schizophrenia.

The brain plasmalogen increaser is produced by a known method, and contains the aforementioned compound and the like as effective components. The brain plasmalogen increaser contains, for example, from 0.1% to 99% by weight, from 1% to 50% by weight, and preferably from 1% to 20% by weight of plasmalogen as an effective component.

The brain plasmalogen increaser according to the present embodiment is administered to humans or animals excluding humans. Preferred examples of the animals include mammals, more specifically, dogs, cats, cows, pigs, horses, sheep, and deer. The administration route of the brain plasmalogen increaser for humans or the like is not limited. The brain plasmalogen increaser is preferably used as an external preparation, an injection solution, an orally administered agent, or the like.

The brain plasmalogen increaser is provided in the form of liquids, tablets, granules, fine granules, powders, tablets, capsules, or the like.

The dose of the brain plasmalogen increaser is appropriately determined based on the age, body weight, symptoms, and the like of the human or other subject to whom the brain plasmalogen increaser is administered. The brain plasmalogen increaser is administered in an effective amount of the aforementioned compound and the like. An effective amount is the amount of the compound and the like necessary to increase plasmalogen in the brain.

As described in detail above, the brain plasmalogen increaser according to the present embodiment can increase plasmalogen in the brain.

In another embodiment, an oral composition for increasing brain plasmalogen including the aforementioned compound and the like is provided. Specific examples of the oral composition for increasing brain plasmalogen include supplements, food compositions, foods and beverages, functional foods, food additives, and the like. The brain plasmalogen increaser may be used as supplements, or may be added to foods and beverages and functional foods.

The forms of the supplements are not limited, and may be any of tablets, powders, granules, capsules, sugar-coated tablets, film formulations, troches, chewable formulations, solutions, emulsions, suspensions, and the like. The supplements may include any component that is normally used for supplements.

"Functional food" means a food or beverage consumed for the purpose of maintaining health, and examples of the functional food include: foods for specified health uses, foods with function claims, and foods with nutrient function claims that are foods with health claims; health foods; dietary supplements; and the like. The functional foods are preferably foods for specified health uses or foods with nutrient function claims that are foods with health claims. In cases of commercialization as a functional food, the oral composition for increasing brain plasmalogen may include various additives for use in foods, such as colorants, preservatives, thickening stabilizers, antioxidants, bleaching agents, antibacterial and antifungal agents, acidulants, sweeteners, seasonings, emulsifiers, fortifiers, agents for production, flavors, and the like.

The functional food may be either a food or beverage, and is not limited as long as the functional food can be orally taken. Examples of the forms of the functional food include beverages, confectionery, processed cereal products, kneaded products, dairy products, and seasonings. Examples of the beverages include nutritional drinks, soft drinks, black tea, and green tea. Examples of the confectionery include candies, cookies, tablets, chewing gums, and jellies. Examples of the processed cereal products include bread, cooked rice, and biscuits. Examples of the kneaded products include sausage, ham, and *kamaboko* that is a boiled fish paste. Examples of the dairy products include butter and yogurt.

The oral composition for increasing brain plasmalogen may be added to foods as food additives. In such a case, examples of the food additives may include pastes, gel-like agents, powders, liquids, suspensions, emulsions, granules, and the like, from the viewpoint of easily adding the food additive to foods.

The oral composition for increasing brain plasmalogen may contain water, vitamins, minerals, organic acids, organic bases, fruit juices, flavors, functional components, food additives, and the like, as long as the oral composition for increasing brain plasmalogen maintains the action of increasing plasmalogen in the brain. The oral composition for increasing brain plasmalogen may be produced by a known method in which components other than the compound and the like are added, as necessary.

The oral composition for increasing brain plasmalogen may be divided into one or more containers such that the daily intake is in accordance with the above-described amount of intake. In such a case, the oral composition for increasing brain plasmalogen is preferably contained in each container in an amount corresponding to the daily intake.

From the viewpoint of the fact that the oral composition for increasing brain plasmalogen is used for increasing plasmalogen in the brain, the oral composition for increasing brain plasmalogen is provided in forms of products that can be distinguished from other products. For example, at least one of the product packaging, instructions, and advertisements for the oral composition for increasing brain plasmalogen indicates that the oral composition for increasing brain plasmalogen has an action for increasing plasmalogen in the brain.

When the oral composition for increasing brain plasmalogen is provided as a food or beverage composition, the oral composition for increasing brain plasmalogen may be offered or sold as a food or beverage with an indication of the application for increasing brain plasmalogen. The application includes health purposes. The act of "indication" includes all acts that inform consumers of the aforementioned application. Any expression that evokes or suggests the aforementioned application is considered an act of "indication", regardless of the purpose, content, object, medium, and the like, of the indication.

The "indication" is preferably made in a manner that allows the consumers to directly recognize the aforementioned application. Specifically, this includes acts such as an act of transferring, delivering, exhibiting for transfer or delivery, or importing food and beverage products or product packaging with the aforementioned application described; an act of exhibiting or distributing advertisements, price lists, or transaction documents relating to the products with the aforementioned application described, or providing information of the content with the aforementioned application described via electromagnetic means such as the Internet.

The "indication" may include indications as health foods, functional foods, enteral nutritional foods, foods for special uses, foods with health claims, foods for specified health uses, foods with nutrient function claims, foods with function claims, quasi-drugs, and the like. Among these, particularly, indications approved under systems for foods for specified health uses, foods with nutrient function claims, or foods with function claims, or similar systems are included. Specifically, examples of the indications include an indication as foods for specified health uses, an indication as conditional foods for specified health uses, an indication of effects on body structure or function, an indication for disease risk reduction, and an indication of functionality based on scientific evidence. More specifically, typical examples thereof include an indication as foods for specified health uses, in particular, an indication of health purposes, and similar indications.

In another aspect of the present embodiment, use of the aforementioned compound and the like for producing the brain plasmalogen increaser or the oral composition for increasing brain plasmalogen is provided. In yet another aspect of the present embodiment, a method of increasing brain plasmalogen including a step of administering the aforementioned compound and the like to a subject is provided. In still another aspect of the present embodiment, the aforementioned compound and the like for the use in increasing brain plasmalogen is provided.

### Examples

### Example 1: Synthesis of Compound KIT-13

KIT-13 was synthesized as a compound according to Formula (I) as follows.

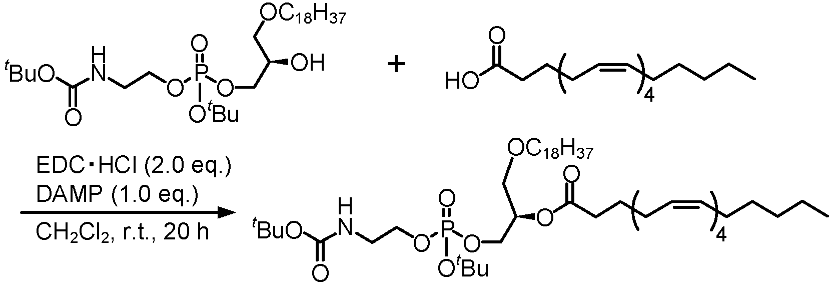

To a solution of tert-butyl(2-((tert-butoxy((R)-2-hydroxy-3-(octadecyloxy)propoxy)phosphoryl)oxy)ethyl)carbamate (187 mg, 0.30 mmol) in dichloromethane (3 mL), EDC hydrochloride (115 mg, 0.60 mmol), DMAP (37 mg, 0.30 mmol), and arachidonic acid (100 mg, 0.33 mmol) were added at room temperature, and the resulting mixture was stirred for 20 hours. The reaction solution was diluted with ethyl acetate, and the resulting dilution was washed with water. The ethyl acetate layer was dried over anhydrous sodium sulfate, and then concentrated under reduced pressure. The residue was purified by column chromatography, to obtain a desired product, (2R)-1-((tert-butoxy(2-((tert-butoxycarbonyl)amino)ethoxy)phosphoryl)oxy)-3-(octadecyloxy)propan-2-yl(5Z,8Z,11Z,14Z)-icosa-5,8,11,14-tetraenoate, as a colorless liquid (260 mg, 95%).

¹H NMR (500 MHz, CDCl₃) δ 5.43-5.31 (m, 8H), 5.16 (quin, J = 4.9 Hz, 1H), 5.16 (br, 1H), 4.21 (m, 1H), 4.14-4.09 (m, 1H), 4.07-4.02 (m, 2H), 3.55 (d, J = 5.3 Hz, 2H), 3.47-3.38 (m, 5.3 Hz), 2.85-2.79 (m, 6H), 2.35 (t, J = 7.6 Hz), 2.12 (q, J = 7.0 Hz, 2H), 2.06 (q, J = 7.1 Hz, 2H), 1.74-1.67 (m, 2H), 1.56-1.54 (m, 2H), 1.502 (s, 9H, one diastereomer), 1.496 (s, 9H, the other diastereomer), 1.39-1.25 (m, 38H), 0.90-0.87 (m, 6H).

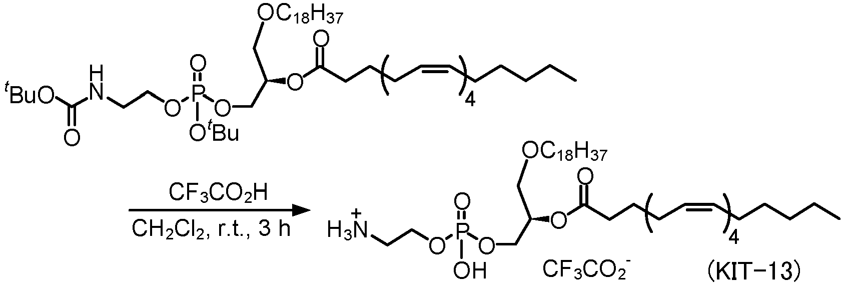

To a solution of (2R)-1-((tert-butoxy(2-((tert-butoxycarbonyl)amino)ethoxy)phosphoryl)oxy)-3-(octadecyloxy)propan-2-yl(5Z,8Z,11Z,14Z)-icosa-5,8,11,14-tetraenoate (260 mg, 0.29 mmol) in dichloromethane (2 mL), 2 mL of trifluoroacetic acid was added at room temperature, and the resulting mixture was stirred for 3 hours. The reaction mixture was concentrated under reduced pressure, to obtain a desired product, 2-(hydroxy((R)-2-(((5Z,8Z,11Z,14Z)-icosa-5,8,11,14-tetraenoyl)oxy)-3-(octadedecyloxy)propoxy)phosphoryl)oxy)ethane-1-ammonium 2,2,2-trifluoroacetate (KIT-13), as a colorless liquid (249 mg, quant.).

¹H NMR (500 MHz, CDCl₃) δ 11.31 (br, 1H), 5.41-5.31 (m, 8H), 5.16-5.15 (m, 1H), 4.17 (br, 2H), 4.07-4.00 (m, 2H), 3.54 (d, J = 4.9 Hz, 2H), 3.45-3.39 (m, 2H), 3.27 (br), 2.84-2.78 (m, 6H), 2.35 (t, J = 7.4 Hz, 2H), 2.10 (q. J = 7.1 Hz, 2H), 2.05 (q. J = 7.2 Hz, 2H), 1.69-1.66 (m, 2H), 1.54-1.51 (m, 2H), 1.39-1.25 (m, 38H), 0.90-0.86 (m, 6H).

### Example 2: Study of Effects of Compound KIT-13 on Brain Plasmalogen

Four-week-old male MeCP2-deficient mice lacking the MeCP2 gene on the X chromosome were used in the test. The MeCP2-deficient mice were divided into two groups: a control group (MeCP2-KO) and a KIT-13 administration group (KIT-13).

For the KIT-13 administration group, KIT-13 was suspended in water by ultrasonication, and administered twice a week by drinking-water administration such that 20 ng/g body weight of KIT-13 was taken. In contrast, the control group was given only water in a similar manner. KIT-13 was administered for three weeks until the mice were seven weeks old, and the cerebral cortex was collected from the brains of the mice.

The collected cerebral cortex was homogenized in a lysate (0.25 M sucrose, 10 mM HEPES-KOH (pH 7.4), 1 mM EDTA, and a protease inhibitor cocktail) to prepare a tissue lysate (25 µg protein). To the tissue lysate in a sample tube, 400 µL of MeOH and 100 µL of internal standard IS (C12 phosphatidylethanolamine (PE) and C12 phosphatidylcholine (PC)) dissolved in chloroform/methanol (C:M=1:2) were added and then mixed. The resulting mixture was transferred to a glass tube. Then, 200 µL of MeOH was added again to the sample tube, and after the same operation, the resulting mixture was transferred to the glass tube. To the sample, 1.2 mL of C/M (1:1) was added, mixed, sonicated for 5 minutes, and left to stand for 60 minutes. Subsequently, 0.625 mL of chloroform was added to the sample and mixed, and then 0.625 mL of MilliQ water was added to the sample and mixed. The sample was centrifuged at 3000 rpm for 5 minutes, and the lower layer was transferred to another glass tube. The remaining upper layer was mixed with 1 mL of chloroform, centrifuged at 3000 rpm for 5 minutes, and combined with the previously collected lower layer. Thereafter, the sample was dried under nitrogen gas, dissolved in MeOH, and analyzed using liquid chromatography-mass spectrometry (LC-MS/MS). The LC-MS/MS conditions are as follows.
LC System: Waters ACQUITY UPLC (trademark) H-Class
MS: Xevo TQ-S micro
MS software: MassLynx V4.1
Data analysis: TargetLynx

### <LC Conditions>

Column: ACQUITY UPLC (trademark) BEH C18 1.7 µm 1.0 × 150 mm
Column temperature: 35°C
Sample solvent: Methanol
Mobile phase:
   (A) Methanol:acetonitrile:water = 2:2:1, 0.1% formic acid, 0.025% ammonia
   (B) 2-propanol, 0.1% formic acid, 0.025% ammonia
Flow rate: 0.07 mL/min
Time Schedule: See Table 1 below.

**Table 1**

| Time (min) | Flow rate (mL/min) | %A | %B | Curve |
|---|---|---|---|---|
| initial | 0.07 | 100 | 0 | |
| 1 | 0.085 | 100 | 0 | 6 |
| 5 | 0.085 | 70 | 30 | 6 |
| 20 | 0.085 | 45 | 55 | 6 |
| 35 | 0.085 | 45 | 55 | 6 |
| 40 | 0.07 | 100 | 0 | 11 |
| 51 | 0.07 | 100 | 0 | |

### <MS Conditions>

Mode: ESI+
Source Voltage
Capillary: 2.0 kV
Cone: 20 V
Source Temperature: 150°C
Desolvation Temperature: 400°C
Source Gas Flow:
   Desolvation: 800 L/hr N₂ Gas
   Cone: 50 L/hr Ar Gas

### Results

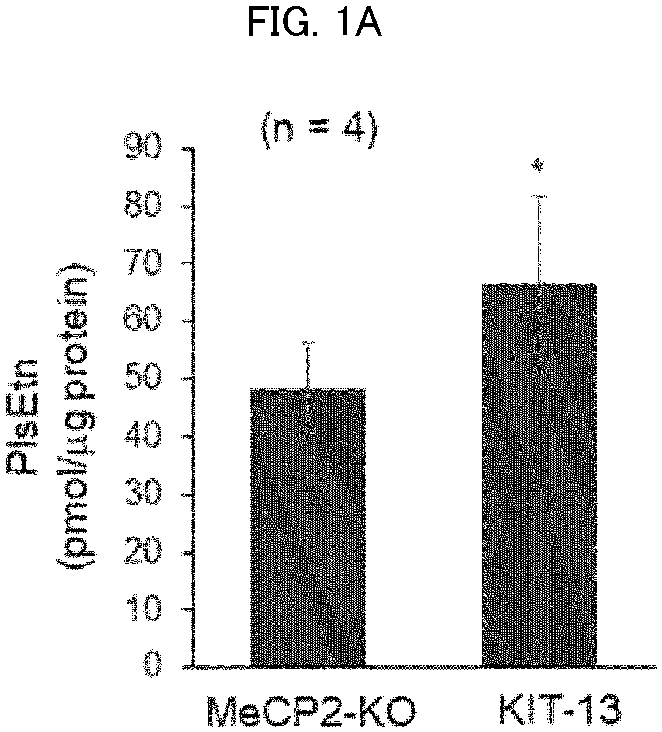

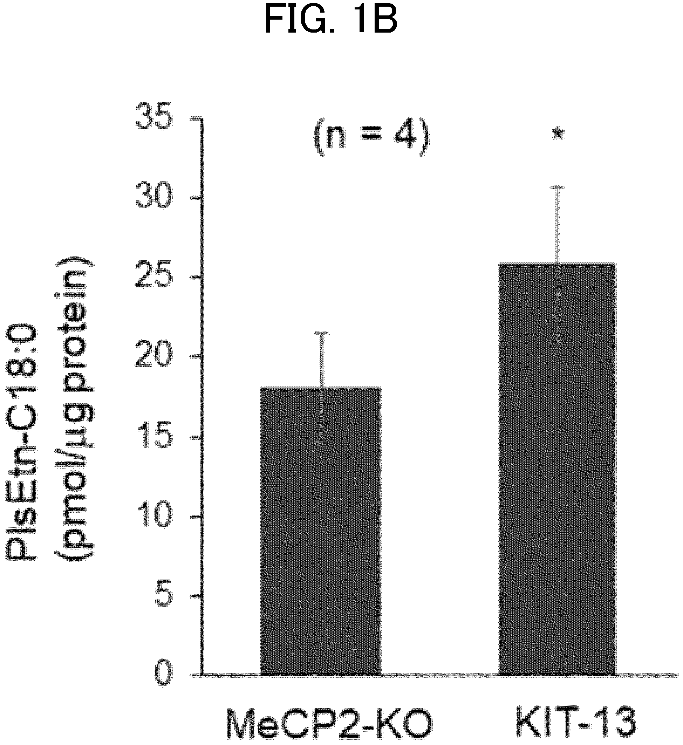

FIG. 1A is a diagram illustrating the concentration of PlsEtn in the tissue lysate. KIT-13 significantly increased PlsEtn in the cerebral cortex (p < 0.05). FIG. 1B is a diagram illustrating the concentration of PlsEtn in which the sn-1 position is a saturated aliphatic hydrocarbon group having 18 carbon atoms (PlsEtn-C18:0), in the tissue lysate. KIT-13 significantly increased PlsEtn-C18:0 in the cerebral cortex (p < 0.05).

### Example 3: Study of Effects of Compound KIT-13 on Cerebral Cortex PlsEtn

Four-week-old wild-type mice (male, C57BL/6J mice, manufactured by the Jackson Laboratory Japan, Inc.) were divided into two groups: a control group and a KIT-13 administration group. The KIT-13 administration group received KIT-13 as with Example 2. The control group was given only water as with Example 2. KIT-13 was administered for four weeks, and the cerebral cortex was collected from the brains of the mice. PlsEtn in the collected cerebral cortex was quantified as with Example 2.

### Results

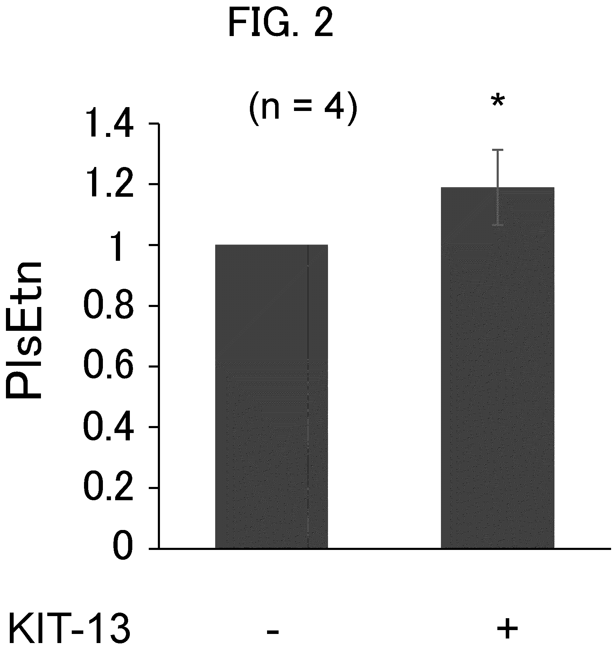

FIG. 2 illustrates relative PlsEtn amount in the cerebral cortex of the KIT-13 administration group (KIT-13; +), taking the PlsEtn amount in the cerebral cortex of the control group (KIT-13; -) as 1. Administration of KIT-13 significantly increased PlsEtn in the cerebral cortex even in wild-type mice.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-185424, filed on November 21, 2022, the entire disclosure of which is incorporated by reference herein.

### Industrial Applicability

The present disclosure is useful for producing pharmaceuticals and oral compositions.

## Claims

1. A brain plasmalogen increaser, comprising: a compound of Formula (I): 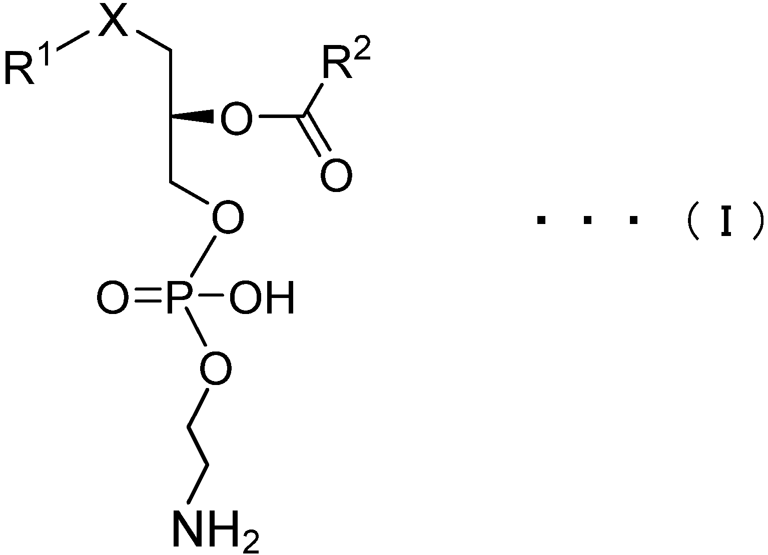 (where, in Formula (I), X represents a carbon atom or an oxygen atom, R¹ represents a saturated aliphatic hydrocarbon group optionally containing a ring structure, and R² represents a saturated or unsaturated aliphatic hydrocarbon group having 10 to 30 carbon atoms, optionally containing a substituent that is an alkyl group having 1 to 4 carbon atoms or an alkoxy group having 1 to 4 carbon atoms),
a racemate of the compound, or a salt of the compound or the racemate.

2. An oral composition for increasing brain plasmalogen, the oral composition comprising:
a compound of Formula (I): 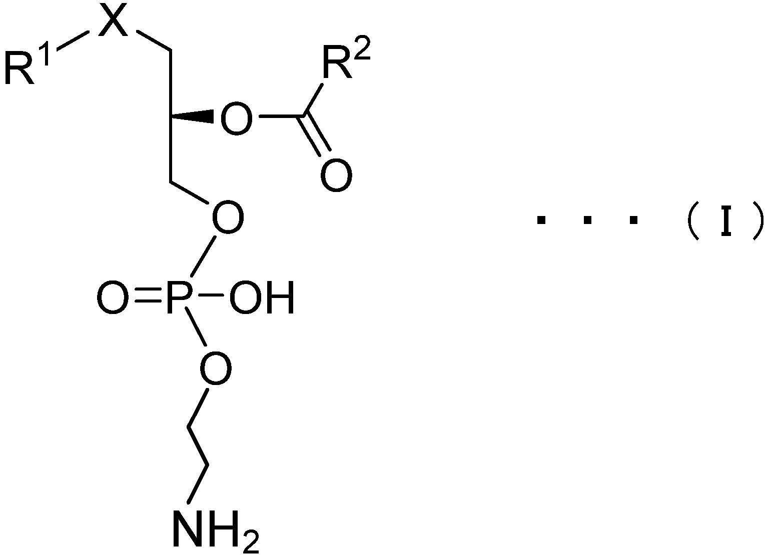 (where, in Formula (I), X represents a carbon atom or an oxygen atom, R¹ represents a saturated aliphatic hydrocarbon group optionally containing a ring structure, and R² represents a saturated or unsaturated aliphatic hydrocarbon group having 10 to 30 carbon atoms, optionally containing a substituent that is an alkyl group having 1 to 4 carbon atoms or an alkoxy group having 1 to 4 carbon atoms),
a racemate of the compound, or a salt of the compound or the racemate.

3. The brain plasmalogen increaser according to claim 1, or the oral composition for increasing brain plasmalogen according to claim 2, wherein
X represents an oxygen atom, R¹ represents an alkyl group having 18 carbon atoms, and R² represents an unsaturated aliphatic hydrocarbon group.

4. The brain plasmalogen increaser or the oral composition for increasing brain plasmalogen according to claim 3, wherein
R², when defined as R²COOH, represents a monovalent group corresponding to arachidonic acid.

5. Use of a compound of Formula (I): 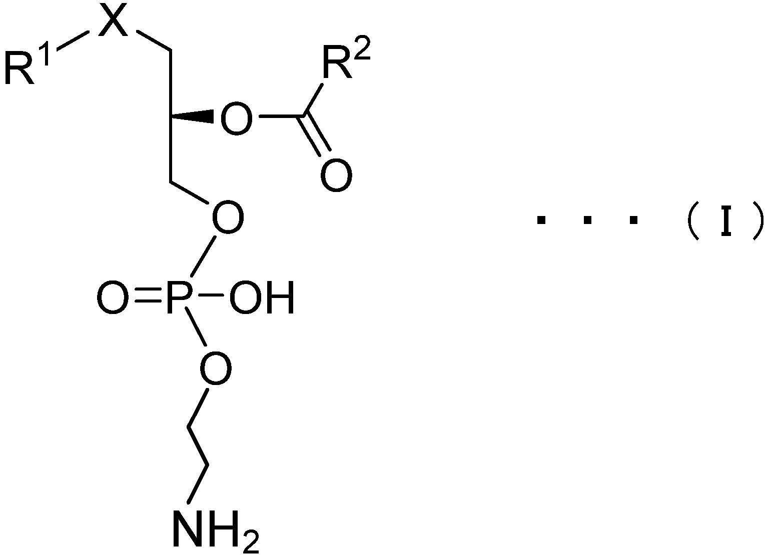 a racemate of the compound, or a salt of the compound or the racemate
for producing a brain plasmalogen increaser or an oral composition for increasing brain plasmalogen (where, in Formula (I), X represents a carbon atom or an oxygen atom, R¹ represents a saturated aliphatic hydrocarbon group optionally containing a ring structure, and R² represents a saturated or unsaturated aliphatic hydrocarbon group having 10 to 30 carbon atoms, optionally containing a substituent that is an alkyl group having 1 to 4 carbon atoms or an alkoxy group having 1 to 4 carbon atoms).
